# EUROPÄISCHE PATENTANMELDUNG

(11) **EP 2 599 462 A1**
(43) Veröffentlichungstag der Anmeldung: **05.06.2013**
(21) Anmeldenummer: 12450049.7
(22) Anmeldetag: 27.11.2012
(51) Int. Cl.: A61F 2/44, A61F 2/46

(54) **Zwischenwirbelimplantat**

(30) Priorität: 29.11.2011 AT 64511 U
(71) Anmelder: Schawrda, Christian, 1130 Wien (AT); Sabitzer, Ronald J., 1060 Wien (AT)
(72) Erfinder: Schawrda, Christian, 1130 Wien (AT); Sabitzer, Ronald J., 1060 Wien (AT)
(74) Vertreter: Miksovsky, Alexander

(57) **Zusammenfassung**

Bei einem Implantat (4) zum Einsetzen zwischen Wirbelkörper (1) der Wirbelsäule, wobei das Implantat (4) wenigstens eine in Richtung benachbarter Wirbelkörper (1) gewandte, vorzugsweise durchgehende Ausnehmung bzw. Durchbrechung (12) aufweist und in einer Draufsicht in eingebauter Lage einen Teilbereich einer Hälfte des Zwischenwirbelraums (3) zwischen zwei benachbarten Wirbelkörpern (1) abdeckt, ist vorgesehen, dass das Implantat (4) an seiner zum Äußeren des Zwischenwirbelraums (3) gewandten Seitenfläche (9) in der Draufsicht eine konvexe Krümmung aufweist, wodurch insbesondere unter Anpassung an die anatomischen Gegebenheiten eine zuverlässigere Abstützung des Implantats (4) in eingebautem Zustand möglich wird.

## Beschreibung

Die vorliegende Erfindung bezieht sich auf ein Implantat zum Einsetzen zwischen Wirbelkörper der Wirbelsäule, wobei das Implantat wenigstens eine in Richtung benachbarter Wirbelkörper gewandte, vorzugsweise durchgehende Ausnehmung bzw. Durchbrechung aufweist und in einer Draufsicht in eingebauter Lage einen Teilbereich einer Hälfte des Zwischenwirbelraums zwischen zwei benachbarten Wirbelkörpern abdeckt.

Ein derartiges Implantat ist beispielsweise der US-PS 4 834 757 zu entnehmen, wobei jeweils im Wesentlichen einen rechteckigen Querschnitt aufweisende Implantate vorgesehen sind, welche posterior zwischen jeweils zwei benachbarten Wirbelkörpern in beiden seitlichen Bereichen bzw. Hälften des Zwischenwirbelraums eingesetzt werden. Nachteilig bei dieser bekannten Ausführungsform, welche üblicherweise als PLIF-Implantat bzw. PLIF-Cage bezeichnet wird, ist insbesondere, dass diese Implantate relativ schmal sind und somit eine schmale Auflagefläche haben. Durch Vorsehen von im Wesentlichen einen rechteckigen Querschnitt aufweisenden, quaderförmigen Strukturen entsprechen derartige Implantate nicht der natürlichen Anatomie der Wirbelkörper bzw. des Zwischenwirbelraums, wobei sie insbesondere im mittleren Teil des Wirbels bzw. Wirbelkörpers anliegen, welcher spongiös und somit nicht so hart ist wie der Rand- bzw. Außenbereich. Derart kann, insbesondere unterstützt durch die rechteckige und somit mit scharfen Kanten bzw. Ecken ausgebildete Form eines derartigen Implantats, dieses in den weicheren Bereich der Wirbelkörper eindringen bzw. einsinken. Darüber hinaus ist insbesondere durch die schmale rechteckige Form ein Verkippen und somit ein Verschieben möglich.

Die vorliegende Erfindung zielt darauf ab, ein Implantat der eingangs genannten Art, welches jeweils einen Teilbereich einer Hälfte eines Wirbelkörpers in eingebauter Lage abdeckt, so dass jeweils zwei Implantate zwischen benachbarten Wirbelkörpern angeordnet sind, dahingehend abzuändern, dass die Nachteile des oben genannten Standes der Technik vermieden bzw. zumindest reduziert werden, und insbesondere ein Implantat zum Einsetzen zwischen Wirbelkörper der Wirbelsäule zur Verfügung zu stellen, mit welchem insbesondere in Anpassung an die anatomischen Gegebenheiten eine zuverlässigere und sichere Abstützung zwischen den benachbarten Wirbelkörpern zur Verfügung gestellt werden kann.

Zur Lösung dieser Aufgaben ist ein Implantat der eingangs genannten Art im Wesentlichen **dadurch gekennzeichnet, dass** das Implantat an seiner zum Äußeren des Zwischenwirbelraums gewandten Seitenfläche in der Draufsicht eine konvexe Krümmung aufweist. Dadurch, dass erfindungsgemäß das Implantat an seiner zum Äußeren des Zwischenwirbelraums gewandten Seitenfläche eine konvexe Krümmung aufweist, ist es im Gegensatz zu dem bekannten Stand der Technik, wo im Wesentlichen rechteckige Außenabmessungen aufweisende Implantate vorgeschlagen werden, mit dem erfindungsgemäßen Implantat möglich, entsprechend den anatomischen Gegebenheiten der Wirbelkörper bzw. des Zwischenwirbelraums ein Implantat in jeweils einer Hälfte der Wirbelkörper anzuordnen, dessen äußere Seiten- bzw. Begrenzungsfläche im Wesentlichen der bogenartigen Außenkontur der Wirbelkörper folgt. Durch Vorsehen einer derartigen gekrümmten bzw. bogenartigen äußeren Seiten- bzw. Begrenzungsfläche wird darüber hinaus die Abstützfläche des Implantats zwischen benachbarten Wirbelkörpern in Anpassung an die anatomischen Gegebenheiten vergrößert und somit die pro Flächeneinheit wirkende bzw. aufzunehmende Kraft entsprechend verringert. Weiters kann durch ein Vermeiden von einen rechteckigen Querschnitt aufweisenden Strukturen ein Einsinken bzw. Einbrechen in dem mittleren Bereich der Wirbelkörper vermieden werden, da durch die außen liegende konvex gekrümmte Seitenfläche eine Abstützung des Implantats in Randbereichen der Wirbelkörper ermöglicht wird, welche eine entsprechend härtere Struktur zur Verfügung stellen.

In weiterer Anpassung an die anatomischen Gegebenheiten und zur Verbesserung der Abstützwirkung an insbesondere eine größere Härte bzw. Festigkeit aufweisenden Randbereichen der Wirbelkörper wird gemäß einer bevorzugten Ausführungsform vorgeschlagen, dass die konvex gekrümmte Seitenfläche wenigstens teilweise dem Verlauf des Apophysenrings nachgebildet ist. Derart kann nicht nur eine zuverlässige Positionierung des Implantats entsprechend der Form bzw. Kontur des Apophysenrings zur Verfügung gestellt werden, wodurch ein Verschieben bzw. Verrutschen verhindert bzw. unterbunden wird, sondern es erfolgt über eine große Fläche auch eine Abstützung des Implantats an den eine größere Härte bzw. Festigkeit aufweisenden Randbereichen der Wirbelkörper.

Zusätzlich wird gemäß einer weiters bevorzugten Ausführungsform vorgeschlagen, dass das Implantat an seinen Enden eine gegenüber dem mittigen Bereich verringerte Höhe aufweist. Dadurch kann der Tatsache Rechnung getragen werden, dass die Wirbelkörper in ihrem mittigen Bereich üblicherweise weiter voneinander beabstandet sind als in den außen liegenden Randbereichen. Durch eine derartige Formgebung des Implantats kann somit die Beweglichkeit der Wirbelsäule bei eingebauten Implantaten entsprechend den natürlichen anatomischen Gegebenheiten aufrecht erhalten bzw. zuverlässig nachgebildet werden.

Für ein einfaches Einführen des Implantats in den Zwischenwirbelraum während der Implantation wird darüber hinaus vorgeschlagen, dass wenigstens ein Ende verringerter Höhe eine abgeflachte oder sich geringfügig konisch zum freien Ende verjüngende Form aufweist, wie dies einer weiters bevorzugten Ausführungsform des erfindungsgemäßen Implantats entspricht.

Neben einer bevorzugt größeren Höhe im mittleren Bereich in Anpassung an die anatomischen Gegebenheiten, wie dies oben erwähnt wurde, ist gemäß einer weiters bevorzugten Ausführungsform vorgesehen, dass das Implantat in seinem mittigen Bereich an wenigstens einer zu einem Wirbelkörper gerichteten Deckfläche mit einer konvexen Wölbung ausgebildet ist. Durch eine derartige zusätzliche konvexe Wölbung an wenigstens einer Deckfläche des Implantats kann den anatomischen Gegebenheiten noch besser Rechnung getragen werden sowie die Beweglichkeit der Wirbelsäule in eingebautem Zustand der Implantate entsprechend unterstützt werden. Eine Anpassung an die teilweise unterschiedlichen Formen bzw. Begrenzungsflächen der Wirbelkörper kann dadurch vorgesehen sein, dass eine Deckfläche im Wesentlichen eine ebene bzw. flache Ausbildung aufweist, während die zweite Deckfläche entsprechend konvex gekrümmt bzw. gewölbt ist. Für einen Einsatz in speziellen Zwischenwirbelräumen kann jedoch erfindungsgemäß vorgesehen sein, dass beide Deckflächen eine konvexe Wölbung bzw. Krümmung aufweisen, welche entsprechend dem Einsatzzweck gegebenenfalls unterschiedliche Krümmungsradien aufweisen.

Für eine Vereinfachung der Positionierung sowie zur Erleichterung des Einführens wird darüber hinaus vorgeschlagen, dass die Enden verringerter Höhe an ihren zu den benachbarten Wirbelkörpern gerichteten Oberflächen mit einer glatten Oberfläche ausgebildet sind, wie dies einer weiters bevorzugten Ausführungsform des erfindungsgemäßen Implantats entspricht.

Ebenfalls zur Unterstützung der anatomisch vorgegebenen Krümmung der Wirbelsäule sowie zur Aufrechterhaltung der Biomechanik derselben wird vorgeschlagen, dass die an benachbarten Wirbelkörpern anliegenden Deckflächen des Implantats miteinander einen spitzen Winkel einschließen, wobei die Höhe des Implantats an dem zum Dornfortsatz gewandten Ende geringer als die Höhe an dem zum Inneren der Wirbelkörper gewandten Ende ist, wie dies einer weiters bevorzugten Ausführungsform des erfindungsgemäßen Implantats entspricht.

Zur Unterstützung einer Verbindung mit dem umliegenden Knochenmaterial bzw. bei einem Verwachsen mit dem Knochenmaterial und auch für eine sichere und zuverlässige Positionierung wird gemäß einer weiters bevorzugten Ausführungsform vorgeschlagen, dass das Implantat in an sich bekannter Weise zumindest an seinen zu den benachbarten Wirbelkörpern gewandten Deckflächen porös und/oder mit einer Profilierung, insbesondere einer Mehrzahl von Erhebungen und Vertiefungen ausgebildet ist.

Für eine weitere Unterstützung einer Verbindung mit dem umgebenden Knochenmaterial bzw. zur Erleichterung eines Verwachsens mit dem umliegenden Material wird darüber hinaus vorgeschlagen, dass in an sich bekannter Weise das Implantat an der zum Inneren der Wirbelkörper gewandten Begrenzungsfläche wenigstens eine Ausnehmung oder Durchbrechung aufweist.

Falls ein Einbringen eines Materials in das Innere des Implantats vorgesehen sein sollte, als auch insbesondere zur Unterstützung einer Verbindung mit umliegendem Material sowie für ein Verwachsen mit demselben, wird gemäß einer weiters bevorzugten Ausführungsform vorgeschlagen, dass die zum Inneren der Wirbelkörper gewandte Ausnehmung bzw. Durchbrechung sich nach außen konisch bzw. trichterförmig erweiternd ausgebildet ist.

Für eine zuverlässige Positionierung des Implantats und/oder eine gegebenenfalls geplante Einbringung eines Materials in das Implantat nach der Positionierung im Zwischenwirbelraum wird gemäß einer weiters bevorzugten Ausführungsform vorgeschlagen, dass das Implantat an wenigstens einem Ende mit einer Durchtrittsöffnung zur Festlegung eines Werkzeugs zum Einsetzen des Implantats und/oder zur Anordnung einer insbesondere rohrförmigen Kanüle zum Einbringen eines Materials in das Innere des Implantats ausgebildet ist. Eine derartige Durchtrittsöffnung, welche beispielsweise mit einem Gewinde versehen sein kann, kann entweder zur Festlegung eines Werkzeugs zur Positionierung des Implantats im Zwischenwirbelraum dienen oder kann zusätzlich oder alternativ zur Anordnung beispielsweise einer rohrförmigen Kanüle zum Einbringen eines Materials in das Innere des Implantats verwendet werden. Es ist somit im Gegensatz zum bekannten Stand der Technik nicht erforderlich, vor dem Einbringen des Implantats in den Zwischenwirbelraum beispielsweise Knochenmasse in dem Hohlraum des Implantats anzuordnen, welche beispielsweise durch große mechanische Beanspruchungen während des Einbringens des Implantats in den Zwischenraum teilweise austreten kann und somit das gewünschte Verwachsen mit dem umgebenen Material nicht entsprechend unterstützen kann. Bei einem erfindungsgemäß vorgesehenen Füllen des Hohlraums des Implantats nach einer Positionierung bzw. Sicherung in der eingebauten Lage im Zwischenwirbelraum gelingt ein zuverlässiges Verfüllen des Hohlraums des Implantats, wodurch nachfolgend ein Verwachsen mit umgebendem Material erleichtert bzw. unterstützt wird. Es kann beispielsweise Knochenzement in das Innere des Hohlraums des Implantats in eingebautem Zustand eingebracht werden, um beispielsweise bei brüchigem Knochenmaterial eine zusätzliche Stützfunktion oder eine zusätzliche Verbindung mit dem umgebenden Knochenmaterial zu ermöglichen.

Die Erfindung wird nachfolgend anhand von in der beiliegenden Zeichnung schematisch dargestellten Ausführungsbeispielen näher erläutert. In dieser zeigen:
Fig. 1 eine schematische teilweise Seitenansicht von zwei benachbarten Wirbelkörpern, zwischen welche jeweils zwei, im Wesentlichen eine Hälfte des Zwischenwirbelraums abdeckende bzw. übergreifende Implantate eingesetzt werden;
Fig. 2 eine schematische Draufsicht auf einen der Wirbelkörper, wobei die Positionierung der erfindungsgemäßen Implantate angedeutet ist;
Fig. 3 eine schematische Rückansicht auf die zwei Wirbelkörper gemäß dem Pfeil III von Fig. 1 und 2, woraus die Positionierung von jeweils zwei Implantaten in dem Zwischenwirbelraum ersichtlich ist; und
Fig. 4 in vergrößertem Maßstab eine Seitenansicht auf eine abgewandelte Ausführungsform eines erfindungsgemäßen Implantats.

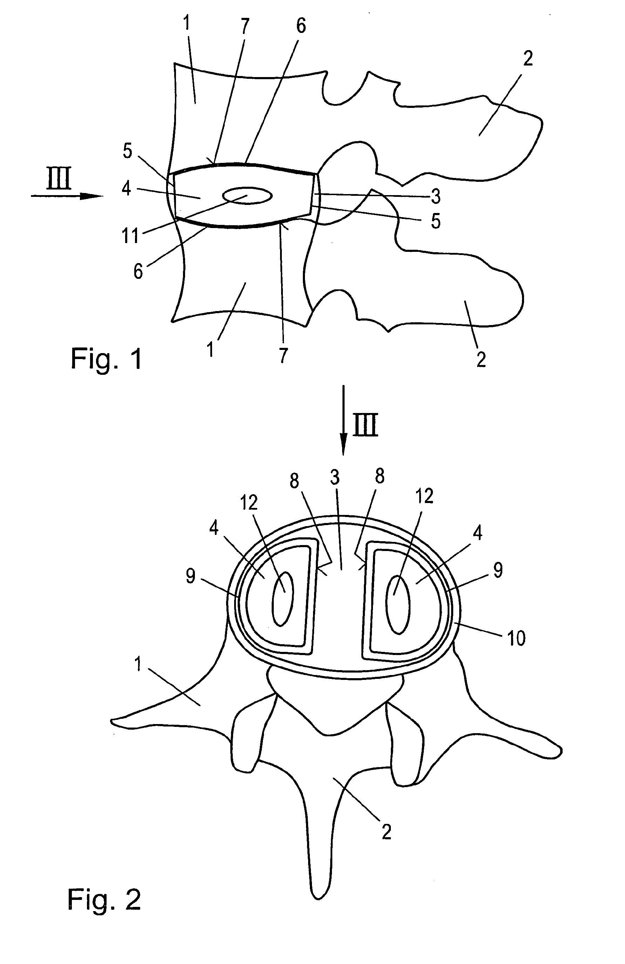

In Fig. 1 sind schematisch mit 1 zwei benachbarte Wirbelkörper bezeichnet, welche jeweils mit Wirbelfortsätzen bzw. Dornfortsätzen 2 ausgebildet sind. In dem Intervertebralraum bzw. Zwischenwirbelraum 3 sind nach einem Entfernen einer Bandscheibe Implantate 4 eingesetzt, wie dies nachfolgend noch im Detail erörtert werden wird.

Aus der Darstellung gemäß Fig. 1 ist ersichtlich, dass das Implantat an seinen Enden 5 jeweils eine gegenüber einem mittleren Bereich 6 verringerte Höhe bzw. Dicke aufweist. Die Deckflächen 6 weisen bei der in Fig. 1 dargestellten Ausführungsform jeweils eine konvexe Wölbung auf, wobei aus Fig. 1 ersichtlich ist, dass die Wölbung bzw. Krümmung der Deckflächen 6 im Wesentlichen den anatomischen Gegebenheiten im Bereich der Begrenzungsflächen 7 der Wirbelkörper entspricht, so dass eine optimale Abstützung der benachbarten Wirbelkörper 1 durch die eingesetzten Implantate 4 möglich wird.

Aus der Darstellung gemäß Fig. 2 ist ersichtlich, dass die Implantate 4 jeweils einen Teilbereich einer Hälfte des Zwischenwirbelraums 3 der benachbarten Wirbelkörper 1 abdecken. Während die Implantate 4 im zu der Mitte gewandten Bereich eine im Wesentlichen ebene Begrenzungsfläche 8 aufweisen, ist an der zum Äußeren der Wirbelkörper 1 gewandten Seite eine eine konvexe Krümmung aufweisende Seiten- bzw. Begrenzungsfläche 9 vorgesehen. Diese Seitenfläche 9 entspricht in ihrer Krümmung im Wesentlichen dem Verlauf des Apophysenrings 10 bzw. gelangt in eingebauter Lage im Wesentlichen in Anlage an den Apophysenring 10. Durch die konvex gekrümmte bzw. gewölbte Seiten- bzw. Begrenzungsfläche 9 wird somit nicht nur eine Vergrößerung der Abstützfläche der Implantate 4 zur Verfügung gestellt, sondern es wird insbesondere eine Abstützung über die konvex gekrümmte Seiten- bzw. Begrenzungsfläche 9 im Bereich des äußeren Randes der Wirbelkörper 1 im Gegensatz zum Stand der Technik möglich, bei welchem im Wesentlichen rechteckige Außenkonturen aufweisende Implantate verwendet wurden, welche entsprechend im mittleren und eine weichere Beschaffenheit aufweisenden Bereich der Wirbelkörper 1 angeordnet wurden.

Aus Fig. 1 und 2 ist ersichtlich, dass die Implantate 4 nicht nur an den nach außen gerichteten Flächen eine Öffnung bzw. Durchbrechung 11 aufweisen, sondern dass insbesondere auch an den zu den benachbarten Wirbelkörpern 1 gewandten Deckflächen 6 wenigstens eine Öffnung bzw. Durchbrechung 12 vorgesehen ist, welche ein Verwachsen mit dem daran angrenzenden Material in eingebauter Lage begünstigt bzw. erleichtert.

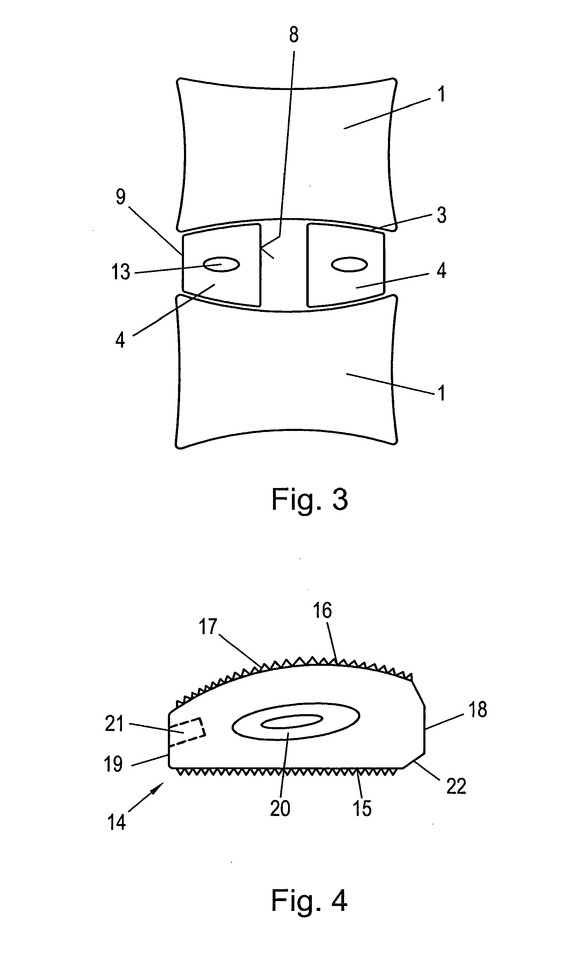

Aus der schematischen Darstellung gemäß Fig. 3 ist darüber hinaus ersichtlich, dass die Implantate 4 jeweils im Wesentlichen symmetrisch angeordnet sind und einen Teilbereich einer Hälfte des Zwischenwirbelraums 3 abdecken.

In Fig. 3 ist darüber hinaus angedeutet, dass die Implantate 4 an einer Endfläche eine Öffnung 13 aufweisen, welche beispielsweise für eine Handhabung des Implantats 4 durch ein Werkzeug bei einem Einsetzen herangezogen werden kann. Alternativ oder zusätzlich kann über die Öffnung 13 beispielsweise durch Verwendung einer Kanüle Material in den inneren Hohlraum des Implantats 4 in eingebauter Lage eingebracht werden. Ein derartiges Material kann ein Verwachsen mit dem umgebenden Knochenmaterial in eingebauter Lage begünstigen oder beispielsweise von einem Knochenzement gebildet sein, um eine Verfestigung zu erzielen.

Aus der Darstellung gemäß Fig. 3 ist weiters ersichtlich, dass die Implantate 4 an ihrer zum Inneren gewandten Seiten- bzw. Begrenzungsfläche 8 eine größere Höhe aufweisen als an der nach außen gerichteten Begrenzungsfläche 9, um derart ebenso den anatomischen Gegebenheiten des Zwischenwirbelraums 3 zu entsprechen.

Während bei einer Ausführungsform gemäß Fig. 1 die Deckflächen 6 jeweils eine konvexe Krümmung in Anpassung an den Zwischenwirbelraum 3 aufweisen, ist bei der in Fig. 4 dargestellten abgewandelten Ausführungsform eines Implantats 14 vorgesehen, dass eine Deckfläche bzw. Begrenzungsfläche 15 im Wesentlichen flach bzw. eben ausgebildet ist, während die zweite Deckfläche bzw. Begrenzungsfläche 16 wiederum eine konvexe Krümmung bzw. Wölbung aufweist. Bei der Darstellung gemäß Fig. 4 ist darüber hinaus angedeutet, dass die Deckflächen 15 und 16 eine Profilierung bzw. poröse Oberfläche 17 aufweisen, um ein Verwachsen mit daran anliegendem Material in eingebauter Lage zu unterstützen bzw. zu begünstigen.

Weiters ist bei der Ausführungsform gemäß Fig. 4 vorgesehen, dass der zum Inneren bzw. zu den Dornfortsätzen 2 gewandte Teilbereich 18 eine größere Höhe als der gegenüberliegende Endbereich 19 aufweist, wobei beide Enden 18 und 19 eine gegenüber dem mittleren Bereich verringerte Höhe aufweisen. Weiters ist in Fig. 4 gezeigt, dass die Endbereiche 18 und 19 im Gegensatz zu den Deckflächen 15 und 16 eine im Wesentlichen glatte Oberfläche aufweisen, um derart ein Einführen zu erleichtern. Darüber hinaus ist der Endbereich 18 mit einer zusätzlichen Verjüngung bzw. Abflachung 22 versehen, um ein Einsetzen bzw. Einführen weiter zu erleichtern bzw. zu unterstützen.

Durch die unterschiedliche Höhe der Endbereiche 18 und 19 und somit einen spitzen Winkel zwischen den Deckflächen 15 und 16 wird eine weitergehende Anpassung an die anatomischen Gegebenheiten und insbesondere die Krümmung der Wirbelsäule erzielt.

In Fig. 4 ist darüber hinaus neben einer Öffnung 20 in einer Seitenfläche eine Durchbrechung bzw. Öffnung 21 angedeutet, welche wiederum beispielsweise für eine Handhabung des Implantats 14 mit einem Werkzeug herangezogen werden kann. Alternativ oder zusätzlich kann insbesondere in eingebauter Lage über die Öffnung 21 beispielsweise durch eine nicht näher dargestellte Kanüle ein Füllen des Hohlraums des Implantats 14 durchgeführt werden, um ein nachfolgendes Verwachsen des Implantats 14 zu begünstigen bzw. zu unterstützen.

Bei der Öffnung bzw. Durchbrechung 20 in der Seitenfläche des Implantats 14 ist darüber hinaus angedeutet, dass sich die Öffnung trichterförmig bzw. konisch erweitert.

## Patentansprüche

1. Implantat zum Einsetzen zwischen Wirbelkörper (1) der Wirbelsäule, wobei das Implantat (4, 14) wenigstens eine in Richtung benachbarter Wirbelkörper (1) gewandte, vorzugsweise durchgehende Ausnehmung bzw. Durchbrechung (12) aufweist und in einer Draufsicht in eingebauter Lage einen Teilbereich einer Hälfte des Zwischenwirbelraums (3) zwischen zwei benachbarten Wirbelkörpern (1) abdeckt, **dadurch gekennzeichnet, dass** das Implantat (4, 14) an seiner zum Äußeren des Zwischenwirbelraums (3) gewandten Seitenfläche (9) in der Draufsicht eine konvexe Krümmung aufweist.

2. Implantat nach Anspruch 1, **dadurch gekennzeichnet, dass** die konvex gekrümmte Seitenfläche (9) wenigstens teilweise dem Verlauf des Apophysenrings (10) nachgebildet ist.

3. Implantat nach Anspruch 1 oder 2, **dadurch gekennzeichnet, dass** das Implantat (4, 14) an seinen Enden (5, 18, 19) eine gegenüber dem mittigen Bereich verringerte Höhe aufweist.

4. Implantat nach Anspruch 3, **dadurch gekennzeichnet, dass** wenigstens ein Ende (18) verringerter Höhe eine abgeflachte oder sich geringfügig konisch zum freien Ende verjüngende Form (22) aufweist.

5. Implantat nach einem der Ansprüche 1 bis 4, **dadurch gekennzeichnet, dass** das Implantat (4, 14) in seinem mittigen Bereich an wenigstens einer zu einem Wirbelkörper (1) gerichteten Deckfläche (6, 16) mit einer konvexen Wölbung ausgebildet ist.

6. Implantat nach einem der Ansprüche 3 bis 5, **dadurch gekennzeichnet, dass** die Enden (5, 18, 19) verringerter Höhe an ihren zu den benachbarten Wirbelkörpern (1) gerichteten Oberflächen mit einer glatten Oberfläche ausgebildet sind.

7. Implantat nach einem der Ansprüche 1 bis 6, **dadurch gekennzeichnet, dass** die an benachbarten Wirbelkörpern (1) anliegenden Deckflächen (15, 16) des Implantats (14) miteinander einen spitzen Winkel einschließen, wobei die Höhe des Implantats an dem zum Dornfortsatz gewandten Ende (19) geringer als die Höhe an dem zum Inneren der Wirbelkörper gewandten Ende (18) ist.

8. Implantat nach einem der Ansprüche 1 bis 7, **dadurch gekennzeichnet, dass** das Implantat (4, 14) in an sich bekannter Weise zumindest an seinen zu den benachbarten Wirbelkörpern (1) gewandten Deckflächen (6, 15, 16) porös und/oder mit einer Profilierung, insbesondere einer Mehrzahl von Erhebungen und Vertiefungen ausgebildet ist.

9. Implantat nach einem der Ansprüche 1 bis 8, **dadurch gekennzeichnet, dass** in an sich bekannter Weise das Implantat (4, 14) an der zum Inneren der Wirbelkörper (1) gewandten Begrenzungsfläche (8) wenigstens eine Ausnehmung oder Durchbrechung (11, 20) aufweist.

10. Implantat nach Anspruch 9, **dadurch gekennzeichnet, dass** die zum Inneren der Wirbelkörper (1) gewandte Ausnehmung bzw. Durchbrechung (20) sich nach außen konisch bzw. trichterförmig erweiternd ausgebildet ist.

11. Implantat nach einem der Ansprüche 1 bis 10, **dadurch gekennzeichnet, dass** das Implantat (14) an wenigstens einem Ende (19) mit einer Durchtrittsöffnung (21) zur Festlegung eines Werkzeugs zum Einsetzen des Implantats (14) und/oder zur Anordnung einer insbesondere rohrförmigen Kanüle zum Einbringen eines Materials in das Innere des Implantats ausgebildet ist.
